# Europäisches Patentamt
# European Patent Office
# Office européen des brevets

(19)

(11) Publication number: **0 016 481**
**B1**

(12) **EUROPEAN PATENT SPECIFICATION**

(45) Date of publication of patent specification: **11.05.83**

(21) Application number: **80200124.8**

(22) Date of filing: **14.02.80**

(51) Int. Cl.³: **C 07 C 79/10,**
**C 07 C 37/045,**
**C 07 C 39/10**

(54) **Process for desensitizing 2,4,6-trinitrotoluene, solutions thus obtained and their application for preparing phloroglucinol.**

(30) Priority: **21.02.79 NL 7901342**

(43) Date of publication of application:
**01.10.80 Bulletin 80/20**

(45) Publication of the grant of the patent:
**11.05.83 Bulletin 83/19**

(84) Designated Contracting States:
**BE CH DE FR GB IT LU NL SE**

(56) References cited:
**DE - A - 2 308 430**

**INDUSTRIAL AND ENGINEERING CHEMISTRY, vol. 42, (1950) M. L. KASTENS et al. "TNT into Phloroglucinol", pages 402—413**

(73) Proprietor: **Océ-Andeno B.V.**
**Grubbenvorsterweg 8**
**NL-5928 NX Venlo (NL)**

(72) Inventor: **Hendrickx, Andreas Joseph Johannes**
**Schubertstraat 31**
**Venlo (NL)**

(74) Representative: **Kremer, Robert A.M., Drs. et al,**
**Océ-Nederland B.V. Patents & Information**
**Postbus 101**
**NL-5900 MA Venlo (NL)**

Process for desensitizing 2,4,6-trinitrotoluene, solutions thus obtained and their application for preparing phloroglucinol

The present invention relates to a process for desensitizing the explosive 2,4,6-trinitrotoluene, hereinafter abbreviated to TNT. The invention also relates to solutions of TNT in very strong sulfuric acid as well as to their application for the preparation of phloroglucinol.

Desensitizing is understood to mean here the minimization of the sensitivity of an explosive compound to heat and shock without it costing the compound's explosive force. The purpose of desensitization is a safer handling of the explosive compound.

Desensitization of explosive compounds, especially of high-explosives, is already known, Urbanski and Galas, for example mention in C.r. 209 (1939) 558 the addition of non-explosive liquids, such as chloroform, n-heptane, acetone, $H_2O$ and glycerin, while the French Patent Specification 863 154 describes how the shock sensitivity of explosive compounds, including TNT, can be diminished by mixing or enveloping the explosive compound with a waxy or fatty material, such as paraffin wax or a stereate.

The French Patent Specifications 2 102 642 and 2 102 815 describe a process of desensitization in which the granular explosive compound is enveloped with a layer of natural or synthetic wax of which the melting point ranges preferably between 60° and 72°C.

Also the German Patent Application 2 308 430, laid open for public inspection, relates to a way of desensitizing, in which the granular high-explosive substance is coated with a layer of wax, but then the grains thus coated are dried and subsequently tempered.

The German Patent Application 2 349 640, laid open for public inspection, cites mono-, di- and trinitro-derivatives of benzene, toluene, xylene, etc. as examples of desensitizing agents for gelatinous explosive compounds. In other words: here, TNT itself is proposed as a desensitizing agent.

All the methods of desensitization mentioned above have the disadvantage that the desensitizing effect is insufficient, as will be further demonstrated below. Moreover, in those desensitizing methods only the application of the desensitized compound as an explosive has been considered. In that case the presence of or envelopment with a comparatively small amount of an inert substance is not inconvenient. In the present case, however, where the TNT is used as a starting material for the preparation of phloroglucinol, the presence of inert substances or the envelopment with layers of wax is highly inconvenient, because these must be removed before, the desensitizing effect thus being lost at the same time.

The object of the present invention is to provide a desensitizing process that does not present the drawbacks mentioned above.

Consequently, the invention relates to a process for desensitizing the explosive TNT, in which there is prepared a solution of TNT in oleum of which the strength (= amount of $SO_3$ dissolved therein) may vary from 10 to 40% (by weight).

Though every gramme of TNT dissolved in oleum is desensitized, desensitizing is only of some practical importance, of course, if at least a certain amount of TNT has been dissolved. Here, an amount of 80 g per litre oleum 10% is chosen as a practical lower limit. The upper limit is determined by the desired degree of densensitization, for the more TNT is dissolved, the more quickly the TNT will crystallize out of the solution — and crystallized TNT is no longer in a desensitized state. As an upper limit an amount of 600 g of TNT per litre oleum 40% is chosen.

It is advantageous to start from the commercially available oleum in which 20 to 25% by weight of $SO_3$ (in short: oleum 20—25%) has been dissolved and preferably the amount of TNT to be dissolved in it ranges from 320 to 500, more particularly from 400 to 480 g per litre oleum.

When a solution of 480 g of TNT per litre oleum 20—25% is stored for 5 x 24 hours at 0°C, the TNT will not crystallize out, even not if the solution is injected with crystalline TNT.

In a solution of 400 g of TNT per litre oleum 20—25%, crystallization will not even occur at —10°C after the solution is allowed to stand for 5 x 24 hours, nor does it when TNT seed crystals are added to this solution.

The desensitizing effect of oleum, as well as that of other desensitizing means, on the explosiveness of TNT when subjected to shock, friction and heat, is evident from the measuring results shown in the table. The values for the sensitivity to shock, friction and heat have been determined according to the statutory methods applied in defining the dangerous materials classes for the transportation of dangerous substances. The said methods are assumed to be known to those skilled in the art. The table also includes the requirements desensitized TNT must meet in order to have been desensitized such as to fall beyond the class to which the transport regulations for substances involving explosion hazard are applicable.

TABLE

| | Sensitivity to shock (in kgm) | Sensitivity to friction (in kgf) | Sensitivity to heat (in mm) |
|---|---|---|---|
| TNT pure | 2.4 | >36 | 5 to 8 |
| Requirements in transport regulations for dangerous substances | >5 | >36 | <1 |
| TNT/talc powder | | | |
| wt/wt   80/20 | 2.5 | >36 | 4 to 6 |
|            70/30 | 3 | >36 | 2 |
| TNT/paraffin wax | | | |
| wt/wt   80/20 | 3.9 | >36 | 3 |
|            70/30 | 6.3 | >36 | 1.5 |
|            60/40 | 7.3 | >36 | 2 |
|            40/60 | – | >36 | >1 |
| TNT/Nibren* wax | | | |
| wt/wt   80/20 | 9.4 | >36 | 3 |
| TNT/kieselguhr | | | |
| wt/wt   80/20 | – | – | 2 |
|            60/40 | 0.5 | – | 0 |
| TNT/oleum 20% 600 g/L | >120 | >36 | <1 |

*Nibren = mixture of tetrachloronaphthalene

From the table it follows that in respect of its sensitivity to heat, TNT is properly desensitized with kieselguhr but, on the other hand, becomes more sensitive to shock, whereas with paraffin wax it is possible, indeed, to satisfy the requirements in respect of the sensitivity to shock but not so in respect of the sensitivity to heat. None of the said desensitizing means talc powder, paraffin wax, Nibren wax or kieselguhr is capable of desensitizing TNT in a sufficient degree.

The table shows the superb desensitizing effect of oleum: with one litre of oleum 20% it is possible to desensitize 600 g of TNT so that it loses its nature of being a dangerous explosive compound.

It must be added that solutions of TNT in concentrated sulphuric acid are already known.

In the production of phloroglucinol from TNT, such as in the large-scale production (described in Industrial and Engineering Chemistry, vol. 42 (1950) 402—413), there is started with the preparation of a solution of TNT in concentrated sulphuric acid. It is to be mentioned that this method for preparing phloroglucinol is the only one that is commercially applied. It is practised already for dozens of years.

Although TNT can also be desensitized by dissolving it in concentrated sulphuric acid (i.e. being at most 100%), this has no practical value because of the too low solubility of TNT in concentrated sulphuric acid. Wetting of the TNT with concentrated sulphuric acid has an insufficient desensitizing effect. In testing the sensitivity to heat of, for example, mixtures of 1,000 g of TNT with 250 to 1,000 ml of concentrated sulphuric acid there are obtained values exceeding 1 mm.

In view of the fact that, in general, in particular however for dangerous explosive compounds such as TNT, the safety and environmental regulations are becoming more and more stringent, the importance of the desensitizing process found is evident. The desensitization now found has considerable consequences for the storage, transportation, handling and processing of TNT.

Thanks to desensitization the TNT can now be stored in areas and buildings, and in ways which, as a rule, are unacceptable for TNT as an explosive compound. For example, as the TNT is present in a dissolved state, it can be stored in tanks and pumped.

To the transportation it applies that if the TNT has been desensitized according to the invention, the carriage may be effected by tank car, in which case the transportation is placed in another, lower dangerous materials class, with all its attendant advantages.

As solutions of TNT in oleum of 10% and higher are new, the invention in view of its importance as outlined above, is also directed on that kind of solutions.

Consequently, the invention also relates to solutions of TNT in very strong sulphuric acid, in which per litre oleum, whose strength may vary from 10 to 40%, at least 80 and at most 600 g of TNT are dissolved, the amount being dependent on the strength chosen for the oleum.

Solutions containing at least 320 g and at most 500 g, more particularly 400 to 480 g of TNT per litre oleum of 20—25%, are preferred.

An additional, very important advantage of the TNT solutions according to the invention lies in that they can be employed as such for the large-scale manufacture of phloroglucinol.

Consequently, the invention is also directed on a process for the preparation of phloroglucinol from TNT, in which process the TNT, dissolved in concentrated sulphuric acid, is converted by oxidation into 2,4,6- trinitrobenzoic acid, which acid is reduced to 2,4,6-triaminobenzoic acid which in turn is hydrolysed and decarboxylated, the process being characterized in that there is started from TNT which is desensitized according to the invention.
The resulting advantages are many, such as:

— eased transport from yard to reaction vessel

— the prior dissolving of TNT in sulphuric acid is no longer necessary

— until the reaction process the TNT is in the desensitized state

— saving of energy and gain of time.

The saving of energy and gain of time realized are due to the fact that the conversion by oxidation into trinitrobenzoic acid occurs in a sulphuric acid medium of lower than 100%, so that the TNT solutions according to the invention must be diluted. The heat released in that process can serve as the necessary heat of reaction for the oxidation step.

In other words: the warming-up step is omitted, energy and time thus being gained.

The following example serves to illustrate the invention.

250 ml of fuming sulphuric acid, containing 20% by weight of $SO_3$ (oleum 20%) are set stirring while being sealed against moisture. To this solution 100 g of TNT in the form of flakes are added in portions at room temperature. Stirring is continued until all the TNT has been dissolved.

**Claims**

1. Process for desensitizing the explosive 2,4,6-trinitrotoluene (TNT), characterized in that there is prepared a solution of TNT in oleum of which the strength may vary from 10 to 40% (by weight).

2. Process according to claim 1, characterized in that at least 80 and at most 600 g of TNT are dissolved per litre oleum, the amount being dependent on the strength chosen for the oleum.

3. Process according to claim 1 or 2, characterized in that at least 320 and at most 500 g of TNT are dissolved per litre oleum of 20—25%.

4. Process according to any one of the claims 1—3, characterized in that at least 400 and at most 480 g of TNT are dissolved per litre oleum of 20—25%.

5. Solution of 2,4,6-trinitrotoluene (TNT) in very strong sulphuric acid, characterized in that per litre oleum whose strength may vary from 10 to 40%, at least 80 and at most 600 g of TNT have been dissolved, the amount being dependent on the strength of the oleum.

6. Solution according to claim 5, characterized in that at least 320 and at most 500 g of TNT have been dissolved per litre oleum of 20—25%.

7. Solution according to claim 5 or 6, characterized in that at least 400 and at most 480 g of TNT have been dissolved per litre oleum of 20—25%.

8. Process for preparing phloroglucinol from 2,4,6-trinitrotoluene (TNT), in which the TNT, dissolved in concentrated sulphuric acid, is converted by oxidation into 2,4,6-trinitrobenzoic acid, which acid is reduced to 2,4,6-triaminobenzoic acid which in turn is hydrolysed and decarboxylated, the process being characterized in that there is started from TNT which is desensitized according to any one of claims 1 to 4.

**Patentansprüche**

1. Verfahren zur Desensibilisierung des Sprengstoffes 2,4,6-Trinitrotoluol (TNT), dadurch gekennzeichnet, dass man eine Lösung von TNT in Oleum, dessen Stärke zwischen 10 und 40% (gew.) variieren kann, herstellt.

2. Verfahren nach Anspruch 1, dadurch gekennzeichnet, dass man, abhängig von der gewählten Oleumstärke, mindestens 80 und höchstens 600 g TNT pro Liter Oleum auflöst.

3. Verfahren nach Anspruch 1 oder 2, dadurch gekennzeichnet, dass man mindestens 320 und höchstens 500 g TNT pro Liter Oleum von 20—25% auflöst.

4. Verfahren nach Anspruch 1 bis 3, dadurch gekennzeichnet, dass man mindestens 400 und höchstens 480 g TNT pro Liter Oleum von 20—25% auflöst.

5. Lösung von 2,4,6-Trinitrotoluol (TNT) in sehr starker Schwefelsäure, dadurch gekennzeichnet, dass abhängig von der gewählten Oleumstärke, mindestens 80 und höchstens 600 g TNT pro Liter Oleum, dessen Stärke zwischen 10 und 40% variieren kann, aufgelöst worden sind.

6. Lösung nach Anspruch 5, dadurch gekennzeichnet, dass mindestens 320 und höchstens 500 g TNT pro Liter Oleum von 20—25% aufgelöst worden sind.

7. Lösung nach Anspruch 5 oder 6, dadurch gekennzeichnet, dass mindestens 400 und höchstens 480 g TNT pro Liter Oleum von 20—25% aufgelöst worden sind.

8. Verfahren zur Herstellung von Phloroglucin aus 2,4,6-Trinitrotoluol (TNT), wobei man in konzentrierter Schwefelsäure aufgelöstes TNT durch Oxidation zu 2,4,6-Trinitrobenzoesäure umsetzt, diese Säure zu 2,4,6-Triaminobenzoesäure reduziert und letztere hydrolysiert und decarboxyliert, dadurch gekennzeichnet, dass man von TNT, das nach einem der Ansprüche 1 bis 4 desensibilisiert worden ist, ausgeht.

**Revendications**

1. Procédé de désensibilisation de l'explosif 2,4,6-trinitrotoluène (TNT), caractérisé en ce que l'on prépare une solution de TNT dans un oléum dont la concentration peut varier entre 10 et 40% (en poids).

2. Procédé selon la revendication 1, caractérisé en ce qu'au moins 80 et au plus 600 g de TNT sont dissous par litre d'oléum, la quantité dépendant de la concentration choisie de l'oléum.

3. Procédé selon l'une des revendications 1 ou 2, caractérisé en ce qu'au moins 320 et au plus 500 g de TNT sont dissous par litre d'oléum à 20—25%.

4. Procédé selon l'une quelconque des revendications 1 à 3, caractérisé en ce qu'au moins 400 et au plus 480 g de TNT sont dissous par litre d'oléum à 20—25%.

5. Solution de 2,4,6-trinitrotoluène (TNT) dans un acide sulfurique très concentré, caractérisé en ce que par litre d'oléum dont la concentration peut varier de 10 à 40%, au moins 80 et au plus 600 g de TNT ont été dissous, la quantité dépendant de la concentration de l'oléum.

6. Solution selon la revendication 5, caractérisée en ce qu'au moins 320 et au plus 500 g de TNT ont été dissous par litre d'oléum à 20—25%.

7. Solution selon l'une des revendications 5 ou 6, caractérisée en ce qu'au moins 400 et au plus 480 g de TNT ont été dissous par litre d'oléum à 20—25%.

8. Procédé pour préparer du phloroglucinol à partir de 2,4,6-trinitrotoluène (TNT) dans lequel le TNT dissous dans l'acide sulfurique concentré est transformé par oxydation en acide 2,4,6-trinitrobenzoïque, cet acide est réduit en acide 2,4,6-triaminobenzoïque qui est à son tour hydrolysé et décarboxylé, le procédé étant caractérisé en ce qu'il part de TNT qui est désensibilisé selon l'une quelconque des revendications 1 à 4.